# EUROPEAN PATENT APPLICATION

(11) **EP 3 539 593 A1**
(43) Date of publication of application: **18.09.2019**
(21) Application number: 18161159.1
(22) Date of filing: 12.03.2018
(51) Int. Cl.: A61M 5/178

(54) **DOCKING STATION FOR DRUG DELIVERY DEVICE**

(71) Applicant: TecPharma Licensing AG, 3401 Burgdorf (CH)
(72) Inventor: Schneider, Andreas, 3027 Bern (CH); Gentz, Michael, 3400 Burgdorf (CH); Kühni, Florian, 3007 Bern (CH); Jordi, Christoph, 5000 Aarau (CH)

(57) **Abstract**

The invention relates to a system comprising a docking station (10), a drug delivery device (30) and an electronic module (40) integrated in or attachable to the drug delivery device (30). The docking station (10) is adapted for accommodating the drug delivery device (30) for leveling a temperature of the drug delivery device (30) from a storage temperature towards a drug delivery temperature. The leveling of the temperature of the drug delivery device (30) is driven by a temperature gradient between ambient temperature and temperature of the drug delivery device (30). The system comprises further a monitoring unit for monitoring a temperature of the drug delivery device accommodated in the docking station (10). Furthermore, the docking station (10) comprises a transmission unit (16) for providing electrical energy to the electronic module (40) integrated on or attached to the drug delivery device (30).

## Description

### FIELD OF THE INVENTION

The present invention relates to a docking station adapted to accommodate a drug delivery device for injecting substances or liquids. It departs form a system comprising the docking station, a drug delivery device and an electronic module integrated in or attachable to the drug delivery device. The electronic module is adapted for monitoring of an injection process executed by means of the drug delivery device.

### BACKGROUND OF THE INVENTION

An electronic module integrated in or attachable to a drug delivery device comprises electronic components that need to be supplied with electrical energy. Therefore, the electronic module comprises an energy storage, usually in form of a rechargeable battery. A common used way to recharge this battery is placing the electronic module with the drug delivery device in a docking station.

The patent application US 2017/182258 A1 discloses an electronic module for a disposable injection pen. The electronic module is adapted to detect or indicate times and dosages of insulin injections and the module comprises a rechargeable battery and a wireless charging induction port. The electronic module may be charged and data from the electronic module can be uploaded to a computing device simultaneously. The uploading is automatically started when the electronic module is connected to the computing device.

Besides that, docking stations are known in the art that comprise additionally an active temperature control for heating or cooling the drug delivery device and therefore heating or cooling the drug comprised in the delivery device. For example, the patent application US 2009/163860 A1 discloses an injector comprises a control portion with a multi-function button to permit a user to select between a first and a second fluid and to selectively adjust the flowrate or the flow property of the fluids. The injector comprises a battery, which is recharged by using electromagnetic induction when the injector is positioned within a docking station. Additionally, the docking station includes a temperature control in order to prevent degradation or decomposition of the medication. The temperature is controlled by refrigeration or heating units. Therefore, the injector, vials and other containers in the docking station are maintained at a particular thermal setting or temperature range by active heating or cooling. The docking station is further connected to a hospital's computing network or to the internet. Information about a treatment procedure such as patient information, drug types and dosages injected may be selectively transmitted from the docking station via network to a receiver. Furthermore, the docking station is connected via Bluetooth to the injector.

Another system is disclosed in the patent application WO 2015/067950 A1. The injection system comprises an injector device with a syringe cage configured to hold and discharge a syringe. The syringe cage includes a heating element which is arranged to heat the contents of the syringe to a desired temperature, before the contents of the syringe are expelled. The injector device includes a plurality of independent temperature sensors embedded into a flexible heater element which is in contact with the glass of the syringe barrel. The placing of the injector device in a docking station initiates a control circuitry in the docking station to activate the heater in the injector device. A monitoring circuit monitors the temperature of the syringe. Once at body temperature, the injector device indicates to the user that the device is ready to use by a display on the docking station.

These prior art approaches focus on a docking station with a charging function and on an active heating or cooling of the drug delivery device in order to obtain a target temperature of the drug. This implies that the docking station, the electronic module or in case of the WO 2015/067950 A1 the syringe cage must comprise an active heating and/or a cooling unit. Such active heating or cooling means take up space, require a complex construction with an appropriate power supply and increase thus considerably the costs for the docking station or the electronic module.

### SUMMARY OF THE INVENTION

It is an objective of the invention to provide for user-friendly and cost-effective ways of preparing a drug delivery system. These objectives are achieved by a system and by a method according to the independent claims. Preferred embodiments are evident from the dependent claims.

According to the invention the drug delivery system comprises a docking station, a drug delivery device and an electronic module integrated in or attachable to the drug delivery device. The electronic module is adapted for monitoring of a drug delivery process or event executed by means of the drug delivery device, and
a. the docking station is adapted for accommodating the drug delivery device for leveling or equilibrating a temperature of the drug delivery device from a storage temperature towards a drug delivery temperature;
b. the leveling of the temperature of the drug delivery device is driven by a temperature difference between ambient temperature and a present temperature of the drug delivery device;
c. the drug delivery system comprises a monitoring unit for monitoring a temperature of the drug delivery device accommodated in the docking station and
d. the docking station further comprises a transmission unit for providing electrical energy to the electronic module integrated in or attached to the drug delivery device.

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances.

The term "drug delivery device" refers to a device for delivering, injecting, administering, infusing or dispensing substances and/or liquids from a product container into the skin of a patient. The injection may be, for example, intracutaneous, subcutaneous or intramuscular. The drug delivery device may be a disposable or a reuseable drug delivery device. A disposable drug delivery device is a system, which is used for injecting the substance from a non-refillable and non-exchangeable cartridge. Once the amount of the substance to be injected either in one single or in several successive delivery events has been delivered the disposable drug delivery device is replaced by a new one. The cartridge in the disposable drug delivery device cannot be replaced. In comparison, the cartridge of a reusable drug delivery device may be replaced an arbitrary number of times.

The electronic module may be attachable to the drug delivery device. Such attachable electronic modules are known in the art. They are also termed add-on, smart device, or smart module. In this case, the drug delivery device is preferably a disposable injection device, for example a disposable pen injector.

But the electronic module does not mandatorily have to be a separate device attachable to the drug delivery device. In contrast, the electronic module may be integrated in the drug delivery device meaning that the electronic module forms an integral part of the drug delivery device. Such a drug delivery device may be, for example, a reusable injection pen or a patch injector for attachment to the skin of a patient. The energy storage unit for such drug delivery devices may be charged before use in order to ensure a proper operation since the energy storage unit may discharge during an extended storage period.

Irrespective of whether the electronic module is integrated in or attachable to the drug delivery device, the electronic module is adapted to recognize events and functions of the injection process, such as time of injection, injected drug, set or expelled doses of drug, circumstances of the injection process, and to keep track of doses already applied. The electronic module is further adapted to report the captured injection data to the user. Furthermore, the electronic module may assist the user in real time during the injection process by providing step-by-step instruction on a display on the electronic module or on a mobile user device. Furthermore, the electronic module may forward the recorded injection data to a docking station or analyze the recorded data.

The storage temperature is to be understood as the temperature of the drug during the storage. The storage temperature can also be termed as initial temperature or as starting temperature of the drug. In contrast, the drug delivery temperature is the temperature suitable for delivering, injecting, administrating, infusing or dispensing the drug into the body of the user. The drug delivery temperature is determined by the drug. Commonly, the drug delivery temperature corresponds to the ambient temperature which lies usually in the range between 18 °C and 35°C. Preferably, the temperature tracking is carried out until the temperature of the drug delivery device has reached a threshold and/or resides within a target temperature range.

According to the invention the levelling of the temperature of the drug delivery device is only driven by a temperature difference between ambient temperature and temperature of the drug delivery device. This implies that no specific and no active heating or cooling means in the docking station or in the electronic module operating at a temperature other than ambient temperature are used to change the temperature of the drug delivery device from the storage temperature to the drug delivery temperature. Thus, the docking station and the electronic module are devoid of any active or specific heating or active or specific cooling means for heating or cooling the drug delivery device placed in the docking station.

Heat transfer to or from the delivery device thus takes place by heat conduction from or to the docking station, and/or by heat exchange with a flow of ambient air circulating around the delivery device by natural or forced convection.

There may be a power dissipation or a loss of heat caused by an electronic circuit or by components of the docking station. Furthermore, the docking station may have a certain heat capacity. Such heat loss is considered to be a part of the ambient air. Therefore, a docking station emitting heat due to power dissipation or due to any electrical or mechanical losses is well within the scope of the invention.

In this context, a fan blowing air at ambient temperature towards the delivery device and hence being devoid of a heating element is not considered a heating element and thus well within the scope of the invention. Nevertheless, even such a fan or other externally powered convection supporting means may be omitted in the docking device and in the electronic module as long as a prolonged temperature equilibration time is acceptable.

It is evident for one skilled in the art that the leveling of temperature of the drug delivery device entrains the leveling of the temperature of the drug in a cartridge of the drug delivery device. The temperature of the housing of the drug delivery device is at least indicative of the temperature of the drug in the cartridge, and a monitoring of a behavior of the delivery device temperature may substitute a monitoring of the drug temperature with adequate accuracy.

Usually, the storage temperature is lower than the drug delivery temperature. In this case the temperature of the drug delivery device placed in the docking station increases during a leveling period until the temperature has reached a drug delivery temperature range. Alternatively, if the temperature of the drug is higher than the drug delivery temperature the temperature decreases during the leveling period until it has reached the drug delivery temperature range.

The monitoring unit for monitoring the temperature may be arranged in the docking station. Alternatively, the monitoring unit may be arranged in the electronic module. In this case, data related to the temperature of the drug delivery device may be transferred from the monitoring unit to the docking station.

Since the drug delivery system according to the invention is devoid of any active heating or cooling means the components of the systems e. g. the docking station and the electronic module can be cost-efficiently and simply manufactured. Nevertheless, in case the target drug delivery temperature corresponds to the ambient temperature the system allows to deliver the drug at the correct temperature. For that purpose, the monitoring unit is adapted to determine periodically whether an instantaneous temperature lies within an ambient temperature range or not.

Preferably, the monitoring unit is adapted to determine periodically based on a computed temperature gradient whether the temperature of the drug lies within the ambient temperature range or not. This is possible since the characteristic behavior of the temperature of any element during the leveling in function of time is well known, e. g. at the beginning the rate of change of the temperature is high and becomes smaller after a certain time. In more mathematical terms, the temperature in function of time during a temperature leveling is a saturation function or an exponential approximation to the ambient temperature. Therefore, it can be concluded that if the rate of change reaches a small value compared with the rate of change at the beginning the temperature of the element is in a range of the ambient temperature, e. g. the temperature difference between the element and the ambient temperature became small. Consequently, it is evident that the temperature gradient at a given time point during temperature leveling of the element can be used to determine whether the temperature of the element has reached an ambient temperature range or not.

Besides that due to the transmission unit of the docking station an energy storage of the electronic module can be charged while the drug delivery device is accommodated in the docking station. This ensures that the electronic module has enough electrical energy for a proper function during subsequent use. In a preferred embodiment the docking station is adapted to provide electrical energy to the electronic module and to monitor the temperature of the drug delivery device simultaneously during leveling of the temperature towards the drug delivery temperature. Therefore, the charging of the energy storage unit of the electronic module takes place in parallel and unnoticed by the user in the background. This increases the usability of the system, as the user intervention is limited to seizing the delivery device, attaching the electronic module, placing the device and module in the docking station, and awaiting a single go-forward signal. Between successive injection events, the electronic module may be kept in the docking station, which is this case is only activated by the presence of the delivery device.

In a preferred embodiment the monitoring unit is adapted to monitor a change of temperature based on a comparison of a computed temperature gradient with a reference gradient or with a reference change of temperature. Preferably, the monitoring unit is adapted to compute the temperature gradient based on a measured temperature of the drug delivery device at a first instant of time and on a measured temperature of the drug delivery device at a second instant of time. The measuring of the temperature at an instant of time is to be understood as a sample of the temperature at a certain point of time.

The monitoring based on the comparison of the computed temperature gradient with the reference gradient offers the advantage that the temperature sensor does not have to be calibrated because the absolute accuracy of the temperature sensor is not crucial and a systematic error or deviation may remain without impact. Relevant is the relative accuracy of the sensor, which is virtually higher in a sensor than the absolute accuracy. This allows to use cost-effective sensors.

In a preferred embodiment the docking station comprises a temperature sensor arranged to measure the temperature on an outside of the housing of the drug delivery device. That means the temperature is not measured on the electronic module or inside the housing but directly on a surface of the housing of the drug delivery device placed in the docking station. The housing is easy accessible for mounting a measuring sensor.

Alternatively, the temperature of the delivery device may be measured directly on a surface of the cartridge comprising the drug. To this end, advantage is taken of an inspection window or opening of the housing of the delivery device, through which the cartridge or syringe barrel may be accessed. For this purpose, the temperature sensor may be mounted on a protrusion that fits into the opening and enables a sufficient thermal contact with the cartridge surface

In another embodiment the docking station may comprise more than one temperature sensor. For example, a first temperature sensor may be arranged at the inspection window of the drug delivery device and a second temperature sensor may be arranged inside the docking station. In this case, the first temperature sensor outputs a changing sensor signal due to the leveling of the temperature of the drug delivery device. In contrast, the second temperature sensor spaced apart from the drug delivery device and arranged inside the docking station nearly outputs a constant sensor signal which may be used as reference signal. Therefore, the monitoring of the temperature may be based on these two sensor signals.

Temperature sensors are well known in the art. For example, an infrared temperature sensor, a surface-mount thermistor or a contact sensor based on a NTC resistor may be used for measuring the temperature on the outside of the housing.

Preferably, the temperature sensor is arranged such that the temperature can be measured on an inspection window of the drug delivery device since there is a good thermal coupling between the inspection window and the cartridge comprising the drug. Therefore, an inspection window is an especially suitable position for measuring the temperature.

Each temperature sensor is connected to the monitoring unit which is preferably arranged in the docking station. The monitoring unit processes the signals of the temperature sensor for monitoring the temperature of the drug delivery device.

Since preferably the change of the temperature (the temperature gradient) in function of time is monitored the absolute value of the temperature of the drug does not have to be computed. However, in an alternative embodiment the temperature of the drug in the housing may be computed based on the thermal transmittance. If the thermal transmittance (depending on the heat transfer coefficient and on the thermal conductivity) is known or if the thermal transmittance can be estimated it is sufficient to measure the temperature on the outside of the housing. Subsequently the temperature of the drug in the housing can be computed.

In an alternative embodiment the electronic module comprises the temperature sensor arranged to measure the temperature on the outside of the housing of the drug delivery device. In contrast to the above described embodiment the docking station preferably does not comprise a temperature sensor. If the temperature sensor is arranged in the electronic module the temperature can be easily measured, for example, on an inspection window of the drug delivery device. If the electronic module is integrated in the drug delivery device the temperature may be measured on the cartridge in the drug delivery device.

The electronic module may comprise more than one temperature sensor. For example, a fist temperature sensor may be arranged to measure the temperature of the housing of the drug delivery device and a second temperature sensor may be arranged inside the electronic module in order to measure the constant ambient temperature. As described above the temperature monitoring may be based on a difference between the measuring values of the first and the second temperature sensor.

In a preferred embodiment the monitoring unit is arranged in the docking station. If the temperature sensor is arranged in the docking station as well the sensor signals are directly transmitted from the sensor to the monitoring unit. If the temperature sensor is arranged in the electronic module the sensor signals are transmitted, preferably wirelessly, from the electronic module to the monitoring unit in the docking station.

Alternatively, the monitoring unit may be arranged in the electronic module if the temperature sensor is arranged in the electronic module. In this case the sensor signals are directly processed in the monitoring unit in the electronic module. Subsequently, data related to the temperature of the drug delivery device are transmitted from the electronic module to the docking station. Such data, for example, may be comprise a marker that the temperature of the drug delivery device has reached the delivery temperature. The data may be displayed to the user by the docking station or if the docking station is connected to a network the data may be transmitted further to the network by the docking station.

In a preferred embodiment the monitoring unit of the docking station is adapted to output a signal when the temperature of the drug delivery device has been determined to reach a drug delivery temperature range. Since the value of the temperature gradient of the drug delivery device is indicative for the absolute temperature of the drug delivery device it can be determined by comparing a computed temperature gradient to a reference gradient whether the temperature of the drug delivery device lies within a drug delivery temperature range or not.

The signal indicates the user when the drug delivery device is ready for use, i. e. when the drug has reached a drug delivery temperature range. Therefore, the user does not have to periodically check the temperature of the drug delivery device which again increases the usability.

Preferably, the monitoring unit may output the signal on the docking station. For example, the signal may be an acoustic signal such as a beep sound or a tactile signal. Alternatively or additionally the signal may be an optical signal, for example, activated Light Emitting Diodes (LED) in traffic-light colors or a message displayed on a display on the docking station. In an alternative embodiment the signal may be outputted in the electronic module as acoustic or optical signal on the electronic module.

Preferably, the transmission unit of the docking station comprises an interface adapted to charge an energy storage unit of the electronic module and wherein the interface is additionally adapted to receive drug delivery data from a transfer unit of the electronic module. The data from the electronic module may comprise recorded information about injection process or data about the injection device, the specification of the drug and the use-by date of the drug. Furthermore, if the electronic module comprises a temperature sensor the sensor signals may be transferred from the electronic module via interface to the docking station. The temperature sensor is preferably a NTC temperature sensor.

Additionally, the interface may be adapted to transfer data from the docking station to the transfer unit of the electronic module. This may be advantageous to display information on the electronic module if the docking station does not comprise any display. Such information may be, for example, the temperature of the drug delivery device or a reminder for an upcoming injection event according to a therapy schedule stored in the docking station. Furthermore, if the docking station is connected to a network data from an external sender may be sent to the electronic module and displayed thereon.

Having one interface being used for transmitting electrical energy as well as for transferring data reduces the components of the docking station and allows a space-saving design of the docking station.

The connection between the transmission unit and the transfer unit may be a physical contact or contactless. Preferably, the interface is adapted to establish a contactless connection between the transmission unit of the drug delivery device and the transfer unit of the electronic module. Thus, the electric energy and the transmission of data occurs without a physical contact element. Such a contactless connection may be realized by inductive or capacitive transmission means. Data transfer and energy storage charging may take place simultaneously, and the dual purpose interface may be designed and optimized such that the expected time for data transfer and energy storage charging is approximately equal to the time for thermal equilibration.

In an alternative embodiment the transmission unit comprises a separate interface adapted to receive data from the transfer unit of the electronic module. In this case the transmission unit comprises a first interface for transmitting electrical energy and a second interface adapted to receive data.

In a preferred embodiment the transmission unit comprises a coil for an inductive transmission of electrical energy by an electromagnetic field to the electronic module. Accordingly, the electronic module comprises a coil acting as receiver for the transmitted electrical energy. This allows a contactless charging of an energy storage of the electronic module. Therefore, abrasion or wear on contact elements of the docking station and the electronic module can be avoided.

In another embodiment the electrical energy may be transmitted by a capacitive coupling. In this case the transmission unit comprises a transmitter electrode and the electronic module comprises a receiver electrode, wherein the two electrodes form a capacitor. In another embodiment the electrical energy can be transmitted by an optical coupling wherein the transmission unit comprises a light source, for example, a LED and the electronic module comprising a photodetector like a photodiode or a photovoltaic array for converting the optical power in electrical power.

In a preferred embodiment the electromagnetic field is additionally used for transferring data unidirectional or bidirectional. Preferably, a short range wireless communication or near field communication is used for transferring the data from the electronic module to the docking station or from the docking station to the electronic module.

In an alternative embodiment the electronic module may comprise a RFID transponder, which can be read out by the transmission unit. Instead or additionally the drug delivery device may comprise a RFID transponder. The docking station may comprise a RFID reader. In this case the docking station may read out the RFID transponder of the electronic module or of the drug delivery device to verify the device. For example, the docking station may output a signal if the verified device meets prespecified requirements.

Alternatively, the transmission unit comprises an electrical contact plate and the electronic module comprises a corresponding electronic contact plate for transmitting the electrical energy from the transmission unit to the electronic module.

Advantageously, the docking station comprises a network unit connected to the interface for exchanging data and adapted to connect the docking station to a network for sending data to the network or receiving data from the network. Through the network unit the docking station may be connected by wire or connected wirelessly, for example, to a local area network (LAN), to a wide area network (WAN) or to the internet. Furthermore, the docking station may be connected via internet to a data storage server, for example, to a data cloud. Preferably, the network unit is adapted to provide a connection to the internet.

From the electronic module data may be transferred through the interface to the docking station and locally stored in the docking station and/or further transferred to an external receiver via the network unit. Data received from an external sender may be stored locally in the docking station and/or may be transferred to the interface and further to the electronic module of the drug delivery device.

Therefore, the docking station preferably works as a gateway. Data can be send via docking station and network to an external receiver. The docking station may also act as gateway for other devices such as infusions devices, for example, an infusion pump or for measuring or monitoring devices such as a blood glucose meter or a heart rate monitor. The docking station is adapted to connect these devices via network unit to the network.

With such a connection to a network injection data recorded by the electronic module can be continuously transferred to the docking station and forwarded to an external receiver. Therefore, an external medical caregiver, for example, may have access almost in real time to injection data. Based on the collected injection data a depth analysis of the injection events and injection behavior is thus possible. In reverse, information from an external sender may be received in the docking station and displayed on a display of the docking station or on a display of the electronic module. Such information, for example, may comprise actual information about changes in the therapy schedule or information about the drug in the drug delivery device.

Preferably, the docking station is adapted to send the signal when the temperature of the drug delivery device reaches the drug delivery temperature range via network to an end user device. Therefore, the user does not have to be next to the docking station during the leveling of the temperature of the drug delivery device.

Preferably, the signal is sent via network unit and internet to the end user device, in particular the signal can be sent wirelessly to a mobile user device such as a mobile phone, tablet or laptop which is wirelessly connected to the docking station. The end user device may be connected via internet with the network unit of the docking station or the end user device may be directly connected to the network unit without internet. In the latter case the end user device may be connected to the network unit by a short range or near range wireless communication technology. The signal may be an acoustic, an optical or a tactile feedback signal or a combination thereof.

In a further preferred embodiment the network unit is adapted to exchange wirelessly data with the electronic module. That means the drug delivery device with the electronic module does not have to be placed in the docking station for exchanging data with the docking station.

Data can be sent wirelessly from the electronic module to the docking station and the data can be forwarded optionally through the network unit to a network, preferably to a data cloud. In reverse, data can be received on the electronic module via network unit of the docking station. Therefore, data can be sent wirelessly by the network unit to the electronic module and the data can be displayed thereon during an injection process. Particularly, this is helpful to provide real time step-by-step instructions for the injection process to the user.

The wireless connection can be realized, for example, by a local WLAN or by another short or near range wireless communication technology such as Bluetooth, ZigBee, WiMAX or NFC. Preferably, the network unit is adapted to exchange data by Bluetooth.

Preferably, the docking station comprises a voice processing unit and further comprises a microphone and a loudspeaker both connected to the voice processing unit. The voice processing unit allows voice interaction. Thus, the microphone may record voice commands from the user. The recorded signals are transferred to the voice processing unit where preferably a voice recognition software may convert the voice command in a computer readable command. On the other hand, an output signal of the docking station may be converted in the voice processing unit to a voice output which can be outputted through the loudspeaker to the user.

Such voice interaction allows the user, for example, to request information form the docking station by voice commands. The docking station may output the requested information by a voice output. Such information may be, for example, the current charging state or the current temperature of the drug of the drug delivery device accommodated in the docking station, the type of the drug or information about the therapy schedule stored in the docking station. Furthermore, if the docking station is connected to a network with the network unit the user may start by voice command a data exchange between the electronic module of the drug delivery device and an external receiver or the user may request data from external care giver by a voice interaction with the docking station.

Advantageously, the docking station comprises a display unit being adapted to display upcoming required user actions according to a timetable or a therapy schedule. The information about required user action may be stored in the docking station and displayed according to the timetable at a given time in order to remember the user of the required action. The action may be, for example, an injection process or a measurement of a body parameter. Alternatively, if the docking station is connected to a network the information about the upcoming required user actions may be received from a network, in particular from an external sender via internet. The received data may then displayed on the display unit of the docking station.

Such a displaying of upcoming required user actions according to a timetable may improve the therapy because the user is reminded to upcoming actions and therefore the risk of forgotten user action is considerably reduced.

In an alternative embodiment the docking station may comprise a speaker to output acoustic signals.

In a preferred embodiment the docking station comprises detection means for detecting if a drug delivery device is accommodated in the docking station. Due to the detection means the user does not have to start manually the monitoring unit and the charging of the electronic module. The detection means recognize when the user places a drug delivery device in the docking station and starts the monitoring and the charging.

In order to detect if a drug delivery device is placed in the docking station the detection means, for example, may comprise a switch which is switched on when getting in contact with the housing of the drug delivery device. Alternatively, the detection means may comprise contactless detection means such as an induction field or a light barrier. These detections means have the advantage that the wear of a contact element can be avoided.

The drug delivery device is preferably a pen shaped injection device, in particular, a disposable pen injector. The injection device has preferably an elongate device body defining a longitudinal main device axis with a distal end where an injection needle is located and a proximal end designating the opposite end.

The invention further comprises a method of monitoring a change of temperature of a drug delivery device, the method comprising the steps of
a. Measuring, by a temperature sensor, at a first instant of time the temperature of the drug delivery device and measuring at a second instant of time the temperature of the drug delivery device;
b. Computing, by a processing unit, a first temperature gradient or time derivative based on the measured temperatures;
c. Monitoring, by a monitoring unit, a temperature leveling of the temperature of the drug delivery device based on the computed temperature gradient.

The monitoring may comprise, for example, a comparison of the computed temperature gradient with a reference temperature rate of change or with a reference temperature gradient.

Reverting to temperature gradients bears the advantage that a systematic absolute error or deviation in the readings of the temperature sensor may remain without impact, and that the absolute accuracy of the temperature sensor therefore is not crucial. Less accurate and/less costly sensor may be employed.

Advantageously, the drug delivery device is accommodated in a docking station during the leveling of the temperature of the drug delivery device. Accordingly, the docking station or the electronic module of the drug delivery device may comprise a temperature sensor for measuring the temperature of the drug delivery device. Furthermore, the docking station or the electronic module comprise a processing unit for computing the temperature gradient.

In an alternative embodiment the temperature of the drug delivery device is measured at a third and at a fourth instant of time, and a second or subsequent temperature gradient is computed based on these measurements, wherein the third instant of time may be at least close to, or preferably even equal to, the second instant of time. Subsequently, the monitoring of the temperature leveling is based on a comparison of the first with the second temperature gradient. Preferably, a threshold for a second derivative of the device temperature may serve as a criteria for establishing that a target temperature range has been reached.

Since the change of temperature of the drug delivery device is small when it has reached a ambient temperature range compared to the change of temperature at the beginning of the temperature leveling a small difference between the first and the second temperature gradient is indicative that the temperature leveling is almost completed. Consequently, it can concluded that the temperature of the drug delivery device lies within an ambient temperature range.

Preferably, the estimated remaining time period until the temperature of the drug delivery device reaches a drug delivery temperature range is computed by extrapolation and the remaining time is displayed on a display unit. Thus, the user knows the time when the drug delivery device will be ready for use.

The extrapolation of the remaining time is preferably based on the instantaneous temperature gradients computed from measured temperatures. Furthermore, the extrapolation of the remaining time may be based on the known temperature behavior during temperature leveling period, e. g. the expected exponential temperature curve in function of time during the temperature leveling between the drug delivery device and the ambient temperature.

The remaining time period may continuously updated such that instantaneous remaining time is displayed. The remaining time may be displayed on a display of the docking station. Alternatively or additionally the remaining period may be displayed on a display on the electric module of the drug delivery device or it may be sent to an end user device.

### BRIEF DESCRIPTION OF THE DRAWINGS

The subject-matter of the invention will be explained in more detail in the following text with reference to preferred exemplary embodiments as illustrated in the attached drawings, of which
- Fig. 1: schematically depicts a docking station and an injection pen detachably connected to an electronic module and horizontally accommodated in the docking station;
- Fig. 2: schematically depicts another embodiment of the docking station with a vertical accommodation for the injection pen connected to the electronic module;
- Fig. 3: schematically depicts an embodiment of the docking station for a patch injector;
- Fig. 4: schematically depicts the wireless connections of the docking station.

For consistency, the same reference numerals are used to denote similar elements illustrated throughout the drawings.

### DETAILED DESCRIPTION OF PREFERRED EMBODIMENTS

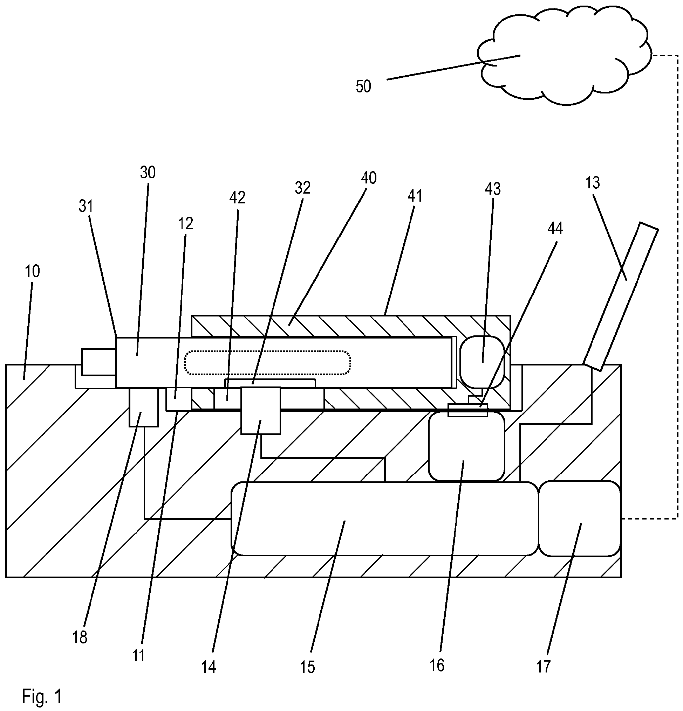

All drawings in the figures 1 - 4 are schematical illustrations. They do not represent all constituent parts and their connections in the docking station, the electronic module and the injection device. The components are depicted in order to show their overall function. In particular, the shown position, connections and size of the components inside the docking station and electronic module may differ in the realized design.

Figure 1 schematically depicts a system with docking station 10, a disposable drug delivery device in the form of a disposable injection pen 30 and an electronic module 40 detachably connected at a proximal end of the injection pen 30. The injection pen 30 with the electronic module 40 are accommodated in a charging tray 11 of the docking station 10. The latter is connected via internet to a data cloud 50.

The injection pen 30 has an elongate housing 31 with a distal end comprising the injection cannula and a proximal end covered by the electronic module 40. The electronic module 40 has an elongate and hollow module housing 41 formed as tubular sleeve forming a cavity adapted to an outer shape of the injection pen housing 31, such that the proximal end of the injection pen 30 may be slidably inserted into the cavity. The module housing 41 has openings 42 that match with windows 32 of the injection pen 30, which in turn allow for visual inspection of a drug comprised in a cartridge in the injection pen 30. The injection pen 30 depicted may be an auto-injector that is triggered by a push-on-skin mechanism. Alternatively, the injection pen 30 may be a variable dose injection device including a proximal release button and a dose selection element in the form of a dosage ring. At least in such configuration the electronic module 40 will take the form of a sleeve shaped passage allowing access to the dose-dialing element and to the release button of the injection pen 30 after insertion into the electronic module 40. The electronic module 40 may also be snap-fit laterally to the injection pen 30 and secured in the attached position by appropriate means.

In the state shown in figure 1 the injection pen 30 is in a connected state with the electronic module 40. In that connected state the proximal end of the injection pen 30 is inserted in the cavity of the electronic module 40 which is adapted for monitoring and recording an injection process executed by an injection mechanism in the injection pen 30. Additionally, the electronic module 40 is adapted to transfer the recorded injection data to the docking station 10. For that purpose the electronic module 40 comprises a transfer unit 44 adapted to receive electrical energy as well to send data. The electronic module 40 comprises further a rechargeable battery for suppling electrical energy to the electronic components 43 in the electronic module 40.

The docking station 10 comprises a housing having a recess 12 in the horizontal top surface. The recess 12 forms the charging tray 11 adapted for accommodating the injection pen 30 with the attached electronic module 40 as shown in figure 1. The contour of the charging tray 11 corresponds to the outer profile of the injection pen 30 with the electronic module 40. That means the recess 12 is deeper in the region of the electronic module 40, such that the longitudinal axis of the injection pen 30 is horizontally aligned when the injection pen 30 is placed in the docking station 10.

The docking station 10 further comprises a display 13, a transmission unit 16 for charging the battery in the electronic module 40, a network unit 17 for connecting the docking station 10 to the internet and a monitoring unit including a temperature sensor 14 and a processing unit 15.

The injection pen 30 with the electronic module 40 being in the charging tray 11 lie within an electromagnetic field provided by the transmission unit 16 of the docking station 10. Thereby, the transmission unit 16 is able to transmit electrical energy to the transfer unit 44 of the electronic module 40 by inductive coupling. Thus, the battery of the electronic module 40 can be charged and the electronic components of the electronic module can be supplied with electrical energy.

In order to establish the required oscillating electromagnetic field the transmission unit 16 comprises an electronic circuit including an oscillator and a coil. The electronic module 40 comprises a corresponding receiver coil for receiving the electrical energy. The electromagnetic field can be further used to transfer data from the electronic module 40 to the docking station 10. In this case, the coil in the electronic module 40 acts as a data sender and the coil in the docking station 10 acts as data receiver. Thus, the transmission unit 16 is adapted to receive data sent by the transfer unit 44 or to send data to the transfer unit 44. Consequently, the transmission unit 16 is adapted to transmit electrical energy as well as adapted to receive and transmit data.

The network unit 17 of the docking station 10 is connected to the transmission unit 16, to the processing unit 15 and is adapted to connect the docking station 10 via internet to the data cloud 50. For that purpose, the network unit 17 comprises an interface that is able to connect the docking station 10 to the internet connection in a premises and additionally an interface for wirelessly connecting the docking station 10 to a WLAN in the premises. Furthermore, the network unit 17 comprises a Bluetooth interface to connect the docking station 10 to mobile phones or tablets via Bluetooth connection. With an initial pairing process the mobile device can be bonded to the docking station 10.

The temperature sensor 14 e.g. in form of an NTC sensor of the docking station 10 is positioned at the bottom of the charging tray 11 and extending into the opening 42 of the electronic module 40 if the injection pen 30 with the electronic module 40 is being placed in the charging tray 11. Thereby, the temperature sensor 14 contacts the inspection window 32 of the injection pen 30 and is thus able to measure the temperature of the inspection window 32. The measuring and the charging of the electronic module can take place simultaneously.

The docking station 10 comprises further an RFID reader 18 to read out an RFID transponder on the injection pen 30.

The RFID reader 18 is located in the charging tray 11 such that it matches the transponder in the injection pen 30 which is located on a distal end of the injection pen 30 where the module housing 41 of the electronic module 40 does not cover the pen housing 31. Due to its position the RFID reader 18 is able to contact directly the transponder. The latter may include information about the type of the injection pen 30, the type of drug and the use-by data of the drug. Within the scope of a supervision of the injection therapy these data may be transferred through the processing unit 15 and the network unit 17 via internet to the data cloud 15 or to an external medical caregiver.

When the user takes the injection pen 30 from the storage place the temperature of the drug in the cartridge in the injection pen 30 is usually not at an appropriate drug delivery temperature since the injection pen is usually stored at a lower temperature, e.g. in the fridge. The delivery temperature is usually in the range of the ambient temperature between 18 °C and 35 °C. In order to level the temperature to the ambient temperature the injection pen 30 is placed in the charging tray 11. The leveling occurs without active heating or cooling and is driven only by the momentary temperature gradient between the ambient temperature and the colder (or hotter) temperature of the drug.

While the injection pen 30 is accommodated in the charging tray 11 the temperature sensor 14 measures periodically the temperature at or near a surface of the inspection window 32 of the pen housing 31, for example, every 10 seconds. The measuring signal from the temperature sensor 14 are sent to the processing unit 15 which displays the temperature on the display 13 stores the measuring data in a data storage of the docking station 10 and/or forwards the measuring data to the network unit 17 for transferring the data to the cloud 15.

The temperature of the drug being colder (or hotter) than the ambient temperature changes strongly at the beginning of the temperature leveling. At the end of the temperature leveling, e.g. when the temperature of the injection pen 30 has reached an ambient temperature range the rate of change of the temperature is very low compared with the rate of change at the beginning of the leveling. Thus, the processing unit 15 can determine if the temperature of the drug has reached an ambient temperature range by comparing a computed temperature gradient with a reference gradient. In other words if the computed temperature gradient is smaller than the reference gradient the temperature of the drug lies within an ambient temperature range.

If the temperature of the drug lies within a predetermined temperature range the processing unit 15 outputs a signal in the form of a text message on the display 13 and/or an activated green LED on the docking station 10 indicating the user that the injection pen 30 is ready for use. In a further embodiment the docking station 10 may additionally output a beep sound in order to inform the user. If the user has a mobile phone or tablet the signal is sent through the network unit 17 via Bluetooth to that user mobile device.

Furthermore, the processing unit 15 computes successively the remaining time until the drug reaches the suitable delivery temperature based on a stored temperature curve. The remaining time is displayed on the display 13 of the docking station 10 and periodically updated.

In another preferred embodiment a first temperature sensor is arranged inside at the proximal end of the electronic module 40 and a second temperature sensor is arranged in the electronic module 40 next to the inspection window 32 of the injection pen 30.

The sensor signal of the first temperature sensor remains more or less constant because the ambient temperature does not change. The second sensor is able to measure the temperature of the inspection window 32 which represents the temperature of the drug. Due to the leveling of the temperature of the drug the sensor signal of the second temperature sensor is changing. In order to monitor the temperature of the drug the first and the second temperature sensor are connected to an electronic circuit forming a voltage divider. That means the temperature sensors in form of NTC resistors are connected in series. After the first sensor follows a center tap and then the second sensor.

The voltage at the center tap is representative for the temperature change because the resistance of the second sensor changes with the change of temperature of the drug while the resistance of the first sensor remains constant. Thus, the voltage at the center tap changes during the leveling of the temperature of the drug and the voltage approaches the half reference voltage applied to the circuit. If the voltage at the center tap is near the reference voltage the resistance of the second sensor corresponds nearly to the (constant) resistance of the first sensor. Thus, the drug has reached the ambient temperature. A signal indicating the end of the temperature leveling may be transferred via electromagnetic field from the transfer unit 44 in the electronic module 40 to the transmission unit 16 and further to the processing unit 15 of the docking station.

During the temperature leveling and as long as the injection pen 30 is accommodated in the charging tray 11 the battery of the electronic module 40 is inductively charged by the transmission unit 16. The user does not have to start the charging manually since the transmission unit 16 detects if an injection pen 30 is placed in the charging tray 11 and automatically starts the charging process. The presence of an injection pen 30 in the charging tray 11 is detected by a change of the electromagnetic field provided by the transmission unit 16.

Besides that the injection data recorded during a previous injection process and stored in the electronic module 40 are transferred via electromagnetic field from the transfer unit 44 in the electronic module 40 to the transmission unit 16 and further to the processing unit 15. Depending on the setting the data are stored in the data storage of the docking station 10 and/or further transferred through the network unit 17 via internet to the data cloud 15 or to any other external receiver, for example, to a medical caregiver. Thus, the docking station 10 works as a gateway transferring data from the electronic module to an external receiver. In reverse, data from the caregiver can be transferred via internet through the network unit 17 to the processing unit 15 and may be displayed on the display 13 of the docking station 10. Instead or additionally the data may be forwarded to the electronic module 40.

Furthermore, a timetable or therapy schedule may be stored in the data storage of the docking station 10. Based on the schedule the processing unit 15 outputs a reminder in form of a text message on the display 13 of the docking station 10 or a beep sound whenever an injection is due. If the user has a mobile device the reminder is sent additionally through the network unit 17 via Bluetooth to the user's mobile device.

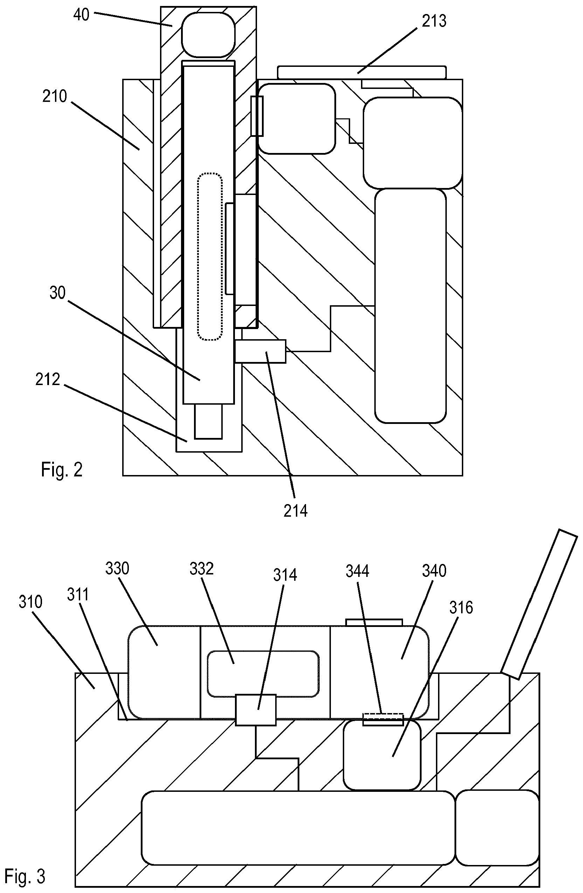

Figure 2 depicts another embodiment of the docking station 210 according to the invention. In contrast to the embodiment shown in figure 1 the docking station 210 comprises a vertical recess 212 adapted to accommodate the injection pen 30 connected to the electronic module 40. Thus, in order to level the temperature of the injection pen 30 and to charge the battery in the electronic module 40 the injection pen 30 has to be inserted with the distal end of the injection pen 30 vertically into the docking station 210.

In contrast to the embodiment of figure 1 the temperature sensor 214 in the embodiment shown in figure 2 is located in the region of the distal end of the injection pen 30 and the temperature is measured on an outside of the housing of the injection pen 30. Furthermore, the display 213 is horizontally aligned on the top surface of the docking station 210. The other components and their function correspond to the ones descripted in figure 1.

In figure 3 a further embodiment of the present invention is shown. In contrast to figure 1 and 2 the drug delivery device is not an injection pen but a patch injector 330 for patching directly onto the skin and for delivery of drugs within a longer time, for example, within 2 to 15 min. The patch injector 330 is square shaped and comprises an integrated electronic module 340 with a rechargeable battery. Bevor use the patch injector 330 is placed in the charging tray 311 of the docking station 310 for temperature leveling and for charging the battery.

The charging tray 311 of the embodiment shown in figure 3 is adapted to the outer contour of the patch injector 330 thereby correctly aligning the patch injector 330 when placed in the charging tray 311. The patch injector 330 may be placed on its narrow side into the charging tray 311. In this position the temperature sensor 314 of the docking station 310 extends through a recess in the patch injector 330 to an inspection window 332 to measure the temperature thereon. In an alternative embodiment the temperature sensor 314 may be arranged in the patch injector 330 and the sensor signals may be transmitted to the docking station 310 via a transfer unit 344.

Similar to the previous embodiments the docking station 310 comprises a transmission unit 316 with electronic circuit including an oscillator and a coil for establishing an electromagnetic field. The patch injector 330 comprises the transfer unit 344 with a corresponding receiver coil. When the patch injector 330 is placed in the charging tray 311 the coil of the docking station 310 matches the coil of the patch injector 330 such that an effective inductive charging of the rechargeable battery in the patch injector 330 is enabled.

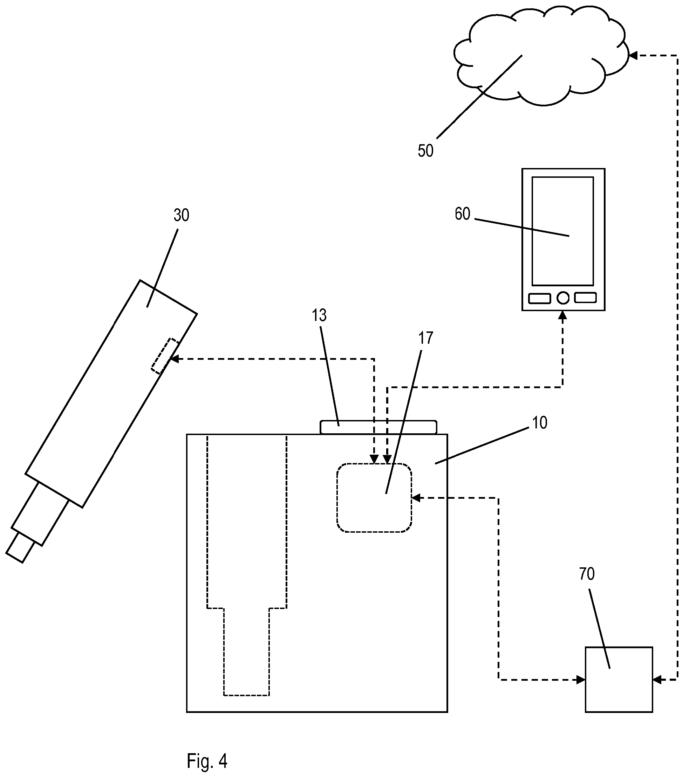

Figure 4 shows schematically the wireless connections of the docking station 10, 210, 310 of the previous shown embodiments. In the figure a pen injector 30 is depicted but the shown connections apply also in a similar manner to a docking station 310 with a patch injector 330 as shown in figure 3. During use of the injection pen 30 or of the patch injector 330 their transfer unit 44, 344 is connected wirelessly to the docking station 10, 210, 310 via Bluetooth. Thus, data recorded by the electronic module 40, 340 can be transferred in real time to the docking station 10, 210, 310 and from there through the network unit 17 via LAN or WLAN to a modem 70 of the premises. Accordingly, the data are transferred from the modem 70 via internet to the data cloud 50 or to an external caregiver. In contrast, the caregiver can send instruction or messages via internet to the docking station 10, 210, 310. The network unit 17 receives the data and forwards it to the processing unit 15 and to the display 13, where the user may see it.

As mentioned above, the data received by the network unit 17 may additionally be forwarded to a user mobile device 60 such as a mobile phone, a tablet or a laptop. Furthermore, signal, alerts or reminders from the docking station 10, 210, 310 may be sent and displayed on the user mobile device 60.

Due to the internet connection the injection process may be automatically controlled. That means the data received from the electronic module 40, 340 or from a possible existing RFID transponder of the injection pen 30 or the patch injector 330 may be sent to an external database where the data are verified. A confirmation whether the injection pen 30, the patch injector 330, the drug, the time of injection or the chosen injection dose is appropriate can be sent to the docking station 10, 310, 310 and displayed thereon while the injection device is still accommodated in the docking station, i.e. in due time for an abort of the planned injection event. This may be realized, for example, by an activated green LED or a text message on the display 13. Of course, such a confirmation may be transferred additionally through the network unit 17 to the electronic module 40 or to a user mobile device.

While the invention has been described in detail in the drawings and foregoing description, such description is to be considered illustrative or exemplary and not restrictive.

### LIST OF REFERENCE NUMERALS

- 10, 210, 310: docking station
- 11, 311: charging tray
- 12,212: recess
- 13: display
- 14, 214, 314: temperature sensor
- 15: processing unit
- 16, 316: transmission unit
- 17: network unit
- 18: RFID reader
- 30: injection pen
- 31: pen housing
- 32,332: windows
- 40, 340: electronic module
- 41: module housing
- 42: openings
- 43: electronic components
- 44, 344: transfer unit
- 50: data cloud
- 60: user mobile device
- 70: modem
- 330: patch injector
- 332: inspection window

## Claims

1. A drug delivery system comprising a docking station (10), a drug delivery device (30) and an electronic module (40) integrated in or attachable to the drug delivery device (30), the electronic module (40) being adapted for monitoring of a drug delivery process executed by means of the drug delivery device (30),
a. wherein the docking station (10) is adapted for accommodating the drug delivery device (30) for leveling a temperature of the drug delivery device (30) from a storage temperature towards a drug delivery temperature,
b. wherein the leveling of the temperature of the drug delivery device (30) is driven by a temperature difference between ambient temperature and a present temperature of the drug delivery device (30),
c. wherein the drug delivery system comprises a monitoring unit for monitoring a temperature of the drug delivery device accommodated in the docking station (10) and
d. wherein the docking station (10) further comprises a transmission unit (16) for providing electrical energy to the electronic module (40) integrated in or attached to the drug delivery device (30).

2. The system according to claim 1, **characterized in that** the monitoring unit is adapted to monitor a change of temperature of the drug delivery device (30) based on a comparison of a computed temperature gradient with a reference gradient.

3. The system according to claim 1 or 2, **characterized in that** the docking station (10) comprises a temperature sensor (14) arranged to measure the temperature on an outside of a housing (31) of the drug delivery device (30).

4. The system according to claim 1 or 2, **characterized in that** the electronic module (40) comprises a temperature sensor (14) arranged to measure the temperature on the outside of the housing (31) of the drug delivery device (30).

5. The system according to claim 3 or 4, **characterized in that** the monitoring unit is arranged in the docking station.

6. The system according to any of claims 1 to 5, **characterized in that** the monitoring unit is adapted to output a signal when the temperature of the drug delivery device (30) reaches a drug delivery temperature range.

7. The system according to any one of claims 1 to 6, **characterized in that** the transmission unit (16) comprises an interface adapted to charge an energy storage unit of the electronic module (40) and wherein the interface is additionally adapted to receive data from a transfer unit (44) of the electronic module (40).

8. The system according to claim 7, **characterized in that** the docking station comprises a network unit (17) connected to the interface for exchanging data and wherein the network unit (17) is adapted to connect the docking station (10) to a network for sending data to the network or receiving data from the network.

9. The system according to claim 8, **characterized in that** the network unit (17) is further adapted for wireless data exchange with the electronic module (40).

10. The system according to claim 1 and 9, **characterized in that** the docking station (10) comprises a voice processing unit, a microphone and a loudspeaker both connected to the voice processing unit.

11. The system according to claim 1 and 10, **characterized in that** the docking station (10) comprises a display unit (13) being adapted to display upcoming required user actions according to a timetable.

12. Method of monitoring a change of temperature of a drug delivery device (30), the method comprising the steps of
a. Measuring, by a temperature sensor (14), at a first instant of time the temperature of the drug delivery device (30) and measuring at a second instant of time the temperature of the drug delivery device (30);
b. Computing, by a processing unit (15), a first temperature gradient based on the measured temperatures;
c. Monitoring, by a monitoring unit, a temperature leveling of the temperature of the drug delivery device from a storage temperature towards a drug delivery temperature based on the computed temperature gradient.

13. Method according to claim 12, **characterized in that** the temperature of the drug delivery device (30) is measured at third and at a fourth instant of time and a second temperature gradient is computed based on these measurements, wherein the monitoring of the temperature leveling is based on a comparison of the first with the second temperature gradient.

14. Method according to claim 12 or 13, **characterized in that** a remaining time period until the temperature of the drug delivery device (30) reaches a drug delivery temperature range is computed by extrapolation and the remaining time is displayed on a display unit (13).

15. Docking station (10) for a drug delivery device (30), wherein the docking station (10)
a. is adapted for accommodating the drug delivery device (30) for leveling a temperature of the drug delivery device (30) from a storage temperature towards a drug delivery temperature driven by a temperature gradient between ambient temperature and the temperature of the drug delivery device (30),
b. comprises a monitoring unit for monitoring a temperature of the drug delivery device (30) accommodated in the docking station (10) and
c. comprises a transmission unit (16) for providing electrical energy to an electronic module (40) integrated in or attached to the drug delivery device (30) accommodated in the docking station (10).
